# EUROPÄISCHE PATENTSCHRIFT

(11) **EP 0 716 851 B1**
(45) Veröffentlichungstag und Bekanntmachung des Hinweises auf die Patenterteilung: **06.08.2003**
(21) Anmeldenummer: 95119285.5
(22) Anmeldetag: 07.12.1995
(51) Int. Cl.: A61K 7/50, A61K 7/48, A61K 33/14

(54) **Badesalz mit einem Gehalt von Magnesiumsalzen und Verfahren zur Herstellung desselben**
Bath salt containing a magnesium salt and its manufacture process
Sel de bain contenant un sel de magnésium et son procédé de fabrication

(30) Priorität: 15.12.1994 DE 4444668
(43) Veröffentlichungstag der Anmeldung: 19.06.1996
(73) Patentinhaber: KRÜGER GmbH & CO. KG, D-51469 Bergisch-Gladbach (DE)
(72) Erfinder: Diezel, Wolfgang, Professor Dr., x (DE); Meric, Ermukan, D-51147 Köln (DE)
(74) Vertreter: Werner, Hans-Karsten, Dr.Dipl.-Chem.

(56) Entgegenhaltungen:
- EP-A- 0 217 975
- DE-A- 2 156 428
- DATABASE WPI Week 9240 Derwent Publications Ltd., London, GB; AN 92-326751 XP002000409 & JP-A-04 230 620 (JINO KOSAN KK)
- STN, Datenbank Liefferant, Karlsruhe, DE XP002000408

## Beschreibung

Gegenstand der vorliegenden Erfindung ist ein Badesalz mit einem Gehalt an Magnesiumsalzen sowie das Verfahren zur Herstellung dieses Badesalzes als rieselfähiges, nicht hygroskopisches Granulat.

Die EP-B-0 439 640 beschreibt Mittel zur äußerlichen Anwendung bei der Therapie entzündlicher Hautkrankheiten sowie zur Abschwächung allergischer entzündlicher Reaktionen der Haut, welche 1 bis 30 Gew.-% Magnesiumchlorid aber keine Calciumsalze sowie Trägersubstanzen enthalten.

Als Mittel zur äußerlichen Anwendung werden neben Salben und Lotionen auch Lösungen, Zahncremes, Kopf- bzw. Körperwaschmittel, Kosmetika und Badezusätze genannt. Salben, Cremes und Lotionen gemäß EP-B-0 439 640 sind bereits mit großem Erfolg in den Handel gebracht worden. Insbesondere bei der Behandlung von Psoriasis (Schuppenflechte) und beim atopischen Ekzem (Neurodermitis) besteht das Bedürfnis als äußerliche Anwendung auch die Sole-Badetherapie durchzuführen.

Zu diesem Zweck sind bereits Badesalzmischungen im Handel, die dem Wasser des Toten Meeres nachgestellt sind. Das Wasser des Toten Meeres enthält über 40 verschiedene Mineralien, welche nur mehr oder weniger für die Wirksamkeit des Gemisches verantwortlich sind. Sie enthalten stets erhebliche Mengen an Calciumsalzen, die jedoch die Wirkung stark abschwächen oder sogar nicht eintreten lassen.

Gemäß EP-B-0 439 640 ist die wichtigste Komponente das Magnesium. Seine Wirksamkeit wird aber durch die Anwesenheit von Calciumionen deutlich verschlechtert oder sogar völlig unterdrückt.

JP-Abstract 3-291 214 beschreibt einen Badzusatz, der einen körpererwärmenden Effekt aufweist, jedoch auch Rostschutz aufweist, so daß die Badeöfen und Badewannen nicht beschädigt werden. Dieser Badzusatz enthält verschiedene Chloride, wie Natriumchlorid, Kaliumchlorid, Magnesiumchlorid oder Calciumchlorid, denen beigemischt werden Natriumcarbonat, Natriumsuccinat, Aluminiumsulfat und Natriumbicarbonat. Hiervon werden 0,4 bis 8 Gew.-% zugemischt auf 1 Gew.-% Chlorid. Hierdurch wird der rostauslösende Effekt der Chloride unterdrückt.

Die DR-PS 670 768 beschreibt ein zur Bereitung von Solbädern geeignetes Badesalz, welches unter Verwendung von Gelatine, Agar-Agar, Tragant, Gummi Acaciae u. dgl. als Bindemittel Mischungen aus Kochsalz und Carnallit enthält, die unter Volumenverminderung miteinander verpreßt werden. Die so erhaltenen Tafeln, Briketts, Platten oder Würfel sind nicht mehr feuchtigkeitsempfindlich, lösen sich jedoch wie gewöhnliches Kochsalz in Wasser auf, ohne Rückstände zu hinterlassen.

JP-Abstract 63-44 515 beschreibt einen Badezusatz, der überwiegend aus Natriumhydrogencarbonat besteht, mit einer bestimmten Korngrößenverteilung. Dieses Gemisch wird granuliert.

Aus Pharmazeutische Technologie, 4. Auflage, 1978, VEB Verlag Volk und Gesundheit, Berlin, Seiten 43 und 44 sowie Seiten 290 bis 293 sind beschrieben die Eigenschaft Hygroskopizität sowie die Herstellung von Granulaten. Es findet sich jedoch kein spezieller Hinweis auf Badesalze.

Aus Fiedler, Lexikon der Hilfsstoffe für Pharmazie, Kosmetik und angrenzende Gebiete, 3. Auflage, 1989, Seite 1328 und Seiten 695 bis 696 ist bekannt, daß Vinylpyrrolidon-Vinylacetat-Copolymere unter der Bezeichnung Kollidon VA 64 unter anderem auch zur Herstellung von Tablettenüberzügen für feuchtigkeitsempfindliche Substanzen einsetzbar ist. Ein Hinweis auf Badesalz findet sich hierin jedoch nicht.

Die Erfindung hat sich die Aufgabe gestellt, ein Badesalz zur Verfügung zu stellen, welches hohe Mengen an Magnesiumchlorid aber keine wesentlichen Mengen an Calciumsalzen enthält.

Diese Aufgabe ist ausgesprochen schwierig zu lösen, da Magnesiumchlorid stark hygroskopisch ist und bereits durch den Zutritt von Luftfeuchtigkeit zerfließt. Es ist somit nicht möglich, das Magnesiumchlorid in reiner Form trocken und rieselfähig zu halten.

Es ist jetzt gelungen, ein Badesalz mit einem Gehalt an Magnesiumsalz zur Verfügung zu stellen, welches dadurch gekennzeichnet ist, daß es als rieselfähiges, nicht hygroskopisches Granulat vorliegt. Dieses Granulat aus Kernen besteht aus mindestens 30 Gew.-% Magnesiumchloridkristallen aber keinen wesentlichen Mengen an Calciumsalzen, wobei die Granulatkörner ummantelt sind mit einem wasserlöslichen aber als Wasserdampfsperre wirkenden Filmüberzug.

Aus den Erfahrungen mit Magnesiumchlorid-Salben ergab sich die weitergehende Aufgabe, diesem Badesalz zusätzlich Vitamin E und/oder ungesättigte Fettsäuren zuzusetzen. Da dies lipophile Substanzen sind, bereitet ihre Zumischung zu einem Badesalz besondere Schwierigkeiten.

Es ist jetzt gelungen, Vitamin E und/oder ungesättigte Fettsäuren in Form von wäßrigen Dispersionen einzusetzen und damit die Magnesiumchloridkristalle zu granulieren.

Um ein rascheres Auflösen des Badesalzes im Badewasser zu erzielen, hat es sich weiterhin als sehr wertvoll erwiesen, dem Badesalz Sprengmittel und/oder Brausehilfsstoffe zuzugeben. Dies ist insbesondere dann möglich, wenn das Sprengmittel und/oder die Brausehilfsstoffe in den Kernen der Granulatkörner vorliegen und das Vitamin E und/oder die ungesättigten Fettsäuren als Filmschicht zwischen den Kernen des Granulats vorliegen. Um die fettigen und öligen Filmschichten des Vitamin E und/oder der ungesättigten Fettsäuren anschließend mit einem wasserlöslichen aber als Wasserdampfsperre wirkenden Film überziehen zu können, empfiehlt es sich, das Granulat vor der Ummantelung abzustäuben. Besonders bewährt hat sich das Abstäuben mit feinteiligem Magnesiumlaktat, welches nach dem Auflösen des Badesalzes weitere Magnesiumionen für die Therapie zur Verfügung stellt.

Als Brausehilfsstoffe und/oder Sprengmittel können auch Gemische von Sprengmitteln mit Brausehilfsmitteln verwendet werden. Als Brausehilfsmittel kommen alle üblichen trockenen Gemische in Frage, die keine Calciumsalze enthalten. Bevorzugt verwendet wird das Gemisch aus Natriumhydrogencarbonat und Zitronensäure. Als Sprengmittel kommen vor allem Stoffe in Frage, die bei Berührung mit Wasser stark quellen. Hierzu gehören getrocknetes Agar-Agar und getrocknetes Guargum. Durch die starke Ausdehnung bei der Berührung mit Wasser helfen diese Sprengmittel, die wirksamen Komponenten rasch im Badewasser aufzulösen.

Da die wichtigsten Komponenten des erfindungsgemäßen Badesalzes Magnesiumchlorid und gegebenenfalls Magnesiumlaktat sind, sollte der Gehalt hiervon vorzugsweise mehr als 50 Gew.-% betragen. Je nach Art und Menge der sonstigen Zusatzstoffe wie Sprengmitteln, Brausehilfsstoffen, Vitamin E und/oder ungesättigten Fettsäuren beträgt somit der Gehalt des erfindungsgemäßen Badesalzes an Magnesiumchlorid 55 bis 95 Gew.-%. Bei Zusatz von Vitamin E und/oder ungesättigten Fettsäuren liegt deren Gehalt zwischen 0,5 und 10 Gew.-%. Mengen von 1,5 Gew.-% Vitamin E und 1 % ungesättigter Fettsäuren haben sich besonders bewährt. Der Gehalt an Sprengmitteln und/oder Brausehilfsmitteln liegt meist im Bereich zwischen 2 und 10 Gew.-%. Ein Gehalt von 5 Gew.-% Brausehilfsmittel hat sich besonders bewährt.

Das Granulieren erfolgt vorzugsweise mit einer wäßrigen Suspension von dl-α-Tocopherolacetat in wasserdispergierbarer Form. Die ungesättigten Fettsäuren werden ebenfalls vorzugsweise als solubilisierte Komplexe mit Hydroalcohol eingesetzt. Als ungesättigte Fettsäuren werden vorzugsweise Linolsäure, Linolensäure und Arachidonsäure verwendet. Prinzipiell kommen aber auch alle anderen einfach oder mehrfach ungesättigten Carbonsäuren in Frage, die sich bei der Behandlung des atopischen Ekzems und Psoriaris bewährt haben.

Die Ummantelung der Granulatkörner mit einem wasserlöslichen aber als Wasserdampfsperre wirkenden Filmüberzug erfolgt beispielsweise mit einem Vinylpyrolidon-vinylacetat-copolymerisat gegebenenfalls unter Zusatz von Hydroxyethylcellulose, Ethylcellulose und/oder pyrogener Kieselsäure. Diese Substanzen lassen sich als alkoholisch-wäßrige Lösung aufsprühen und bilden nach dem Trocknen in Form eines Filmüberzugs einen ausgezeichneten Schutz gegen Feuchtigkeit.

Das erfindungsgemäße Badesalz wird je nach Gehalt an Magnesiumchlorid in Mengen von 200 bis 500 g dem Badewasser (ca. 80 l) zugesetzt. Das Badewasser enthält somit das Magnesiumchlorid in Konzentrationen von weniger als 1 %. Dafür kann es aber über längerer Zeit gleichmäßig von der gesamten Haut aufgenommen werden, so daß es dort seine Wirksamkeit entfalten kann.

Die gute Wirksamkeit des Magnesiumchlorids in dem erfindungsgemäßen Badesalz wurde zunächst getestet an der Hemmung des Crotonöl-induzierten Ohrödems bei Mäusen. Dazu wurden Zubereitungen, die jeweils 10 Gew.-% Magnesiumchlorid enthielten, vergleichend getestet mit aufgelösten Badesalzen, so daß jeweils eine Endkonzentration von ebenfalls 10 Gew.-% Magnesiumchlorid entstand.

In dem Vergleich mit einbezogen wurden handelsübliche Badesalze mit einem Gehalt an Magnesiumsalzen und einen dem Toten Meer entsprechenden Gehalt an Calciumchlorid. Weiterhin verglichen wurden wäßrige Lösungen von Magnesiumchlorid und reines Natriumchlorid.

Bei entzündlichen Hautkrankheiten haben epidermale Langerhans-Zellen (ELZ) eine entscheidende pathogenetische Bedeutung. Diese Zellen zählen zu den Antigen-präsentierenden Zellen. Deren Aktivität wird maßgeblich durch ein Oberflächenenzym bestimmt, eine ATPase. Die Hemmung dieses Enzyms bewirkt eine Hemmung der Funktion der ELZ und damit eine verminderte entzündliche Reaktion auf der Haut. Die Hemmwirkung von Magnesium, dem erfindungsgemäßen Magnesiumbadesalz und anderen vergleichbaren Badesalzen und Kochsalz wurden bestimmt. Die Ergebnisse sind in den nachfolgenden Tabellen 1 und 2 zusammengestellt.

**Tabelle 1**

| Einfluß von Magnesium allein und von verschiedenen Salzlösungen auf ATPase-positive epidermale Langerhans-Zellen (ELZ) in Hautpräparaten von Balb/c-Mäusen | | | |
|---|---|---|---|
| Nr. | ATPase⁺ELZ | n¹⁾ | P^{<}²⁾ |
| 1 Kontrolle | 570 ± 50 | 25 | |
| 2 Magnesiumchlorid (5 %ig) | 375 ± 25 | 5 | 0,05 |
| 3 Magnesiumchlorid (10 %ig) | 120 ± 10 | 5 | 0,001 |
| 4 Magnesiumchlorid-Badesalz (10 %ig) (erfindungsgemäß) | 130 ± 20 | 5 | 0,001 |
| 5 handelsübliches medizinisches Badesalz (10 %ig) enthaltend auch Calciumsalze und Fette | 250 ± 15 | 5 | 0,05 |
| 6 Tropic-Marin-Badesalz (10 %ig) handelsüblich auch Calciumsalze enthaltend | 340 ± 40 | 5 | 0,05 |
| 7 Mineral-Badesalz (10 %ig) handelsüblich auch Calciumsalze enthaltend | 300 ± 30 | 5 | 0,005 |
| 8 NaCl (10 %ig) | 490 ± 50 | 5 | n.s. |

| | | | |
|---|---|---|---|
| 1) Anzahl der Experimente | | | |
| 2) Statistischer Unterschied zu Nr. 1 (Wilcoxon-Test), n.s.: keine statische Differenz | | | |

Epidermis wurde mit den o.g. Salzkonzentrationen 1 h bei 37°C inkubiert, und es wurde nachfolgend die ELZ pro 1 mm² Epidermis bestimmt.

**Tabelle 2**

| Hemmung des Crotonöl-induzierten Ohrödems durch Magnesiumchlorid-haltige Externa (6 Mäuse pro Tiergruppe) | | | | |
|---|---|---|---|---|
| Tiergruppe Nr. | Behandlung | Ödemgewicht¹⁾ (mg) | % Hemmung | P^{<}²⁾ |
| 1 | Alleinige Crotonöl-Behandlung | 16,02 ± 1,60 | | |
| 2 | Crotonöl + MgCl₂-Creme (10 %ig) | 8,02 ± 1,58 | 49 | 0,01 |
| 3 | Crotonöl³⁾ + Badesalz-Creme (MgCl₂-Endkonzentration: 10,0 %) | 7,80 ± 1,72 | 52 | 0,01 |
| 4 | Crotonöl + NaCl-Creme (15 %ig) | 13,46 ± 1,82 | 16 | n.s. |

| | | | | |
|---|---|---|---|---|
| 1) Mittelwert ± Standardabweichung | | | | |
| 2) Wilcoxon-Test: statistischer Unterschied zu Nr. 1, n.s.: keine statistische Differenz | | | | |
| 3) Badesalz-Creme: erfindungsgemäßes Magnesium-Badesalz, inkorporiert in eine Salbengrundlage | | | | |

Erste Beobachtungen bei Patienten mit Neurodermitis und Schuppenflechte mit dem erfindungsgemäßen Badesalz im Badewasser haben sehr gute Erfolge gezeigt. Das erfindungsgemäße Badesalz, insbesondere unter Zusatz von Vitamin E und/oder ungesättigten Fettsäuren, ist somit hervorragend verträglich und wirksam.

Die Herstellung des erfindungsgemäßen Salzes erfolgt dadurch, daß zunächst das Magnesiumchlorid in kristalliner Form mit den Brausehilfsstoffen Natriumhydrogencarbonat und Zitronensäure vermischt wird. Dieses Gemisch wird mit einer wäßrigen Suspension von dl-α-Tocopherolacetat (wasserdispergierbar) und einem Hydroalcohol-solubilisierten Komplex von ungesättigten Fettsäuren (Linolsäure, Linolensäure und Arachidonsäure) auf die Pulvermischung aufgesprüht, wobei dieses granuliert wird. Dieses Material wird mit feinpulvrigen Magnesiumlaktat abgestäubt und anschließend mit einer wäßrigen Lösung von Vinylpyrolidon-vinylacetat-copolymerisat und Hydroxyethylcellulose ummantelt. Nach dem Trocknen hat sich ein Filmüberzug gebildet, der das Granulat gegen Luftfeuchtigkeit schützt und ihm eine hohe Stabilität verleiht. Beim Eingeben ins Wasser löst sich das Badesalz rasch unter leichtem Schäumen auf.

Einige typische Zusammensetzungen sind in den nachfolgenden Beispielen zusammengestellt:

### Beispiel 1

70 Gew.-% Magnesiumchlorid, 10 Gew.-% Magnesiumlactat, 1,5 Gew.- % α-Tocopherolacetat CWS (entsprechend 0,7 Gew.-% α-Tocopherolacetat) und 1,0 Gew.-% Vit F Wasserlöslich (solubilisierter Komplex von ungesättigten Fettsäuren, insbesondere Linolsäure, Linolensäure und Arachidonsäure).

Die Granulatkörner wurden mit vier verschiedenen Filmüberzügen besprüht und getrocknet.

Filmüberzugslösung 1 bestand aus Vinylpyrolidon-Vinylacetat Copolymerisat in wäßriger Lösung in Konzentrationen zwischen 1 und 5 Gew.-%. Dieser Filmüberzug zeigt eine sehr gute Wasserdurchlässigkeit.

Filmüberzugslösung 2 enthielt zusätzlich 0,1 bis 2,5 Gew.-% hydrophob modifizierte Hydroxyethylcellulose.

Filmüberzugslösung 3 enthielt außer Vinylpyrolidon-Vinylacetat Copolymerisat 0,05 bis 2,5 Gew.-% Ethylcellulose. Dieses Gemisch wurde in 75 % wäßrigem Ethanol gelöst und versprüht.

Filmüberzugslösung 4 enthält außer Vinylpyrolidon-Vinylacetat Copolymerisat 0,05 bis 1,5 Gew.-% hydrophobe pyrogene Kieselsäure als wäßrige Suspension.

Die Filmüberzüge 2 bis 4 zeigten gute aber unterschiedliche wasserdampfsperrende Wirkung.

### Beispiel 2

Eine weitere bevorzugte Zubereitung hat die Zusammensetzung

| | |
|---|---|
| Magnesiumchlorid | 55,00 % |
| Magnesiumlactat | 1,00 % |
| Natriumbicarbonat | 10,00 % |
| Vinylpyrolidon-vinylacetat Copolymer | 2,00 % |
| Ethylcellulose | 0,30 % |
| Zitronensäure | 5,00 % |
| Tocopherolacetat | 1,20 % |
| Acharidonsäure, Linolsäure, Linolensäure | 1,00 % |
| Lactose | 24,50 % |

Diese Zubereitung wird wie weiter oben beschrieben hergestellt und ist lagerstabil, in Wasser gut auflösbar, gut wirksam und einwandfrei verträglich.

## Patentansprüche

1. Badesalz mit einem Gehalt an Magnesiumsalzen, **dadurch gekennzeichnet, daß** es als rieselfähiges, nicht hygroskopisches Granulat vorliegt und dieses Granulat aus Kernen besteht, die mindestens 30 Gew.-% Magnesiumchloridkristalle aber keine wesentlichen Mengen an Calciumsalzen enthalten, wobei die Granulatkörner ummantelt sind mit einem wasserlöslichen aber als Wasserdampfsperre wirkenden Filmüberzug.

2. Badesalz gemäß Anspruch 1, **dadurch gekennzeichnet, daß** es zusätzlich Vitamin E und/oder ungesättigte Fettsäuren enthält.

3. Badesalz gemäß Anspruch 1 oder 2, **dadurch gekennzeichnet, daß** es Sprengmittel und/oder Brausehilfsstoffe enthält.

4. Badesalz gemäß Anspruch 2 oder 3, **dadurch gekennzeichnet, daß** das Sprengmittel und/oder die Brausehilfsstoffe in den Kernen der Granulatkörner vorliegen und das Vitamin E und/ oder die ungesättigten Fettsäuren als Filmschicht zwischen den Kernen des Granulats vorliegen.

5. Badesalz gemäß einem der Ansprüche 1 bis 4, **dadurch gekennzeichnet, daß** der Gehalt an Magnesiumchlorid mehr als 50 Gew.-% beträgt.

6. Badesalz gemäß einem der Ansprüche 1 bis 5, **dadurch gekennzeichnet, daß** der Gehalt an Magnesiumchlorid 55 bis 95 Gew.-% beträgt.

7. Badesalz gemäß einem der Ansprüche 2 bis 6, **dadurch gekennzeichnet, daß** der Gehalt an Vitamin E und/oder ungesättigten Fettsäuren 0,5 bis 10 Gew.-% beträgt.

8. Badesalz gemäß einem der Ansprüche 3 bis 7, **dadurch gekennzeichnet, daß** der Gehalt an Sprengmittel und/oder Brausehilfsmittel 2 bis 10 Gew.-% beträgt.

9. Verfahren zur Herstellung von Badesalz gemaß Anspruch 1 **dadurch gekennzeichnet, daß** kristallines Magnesiumchlorid gegebenenfalls im Gemisch mit einem Sprengmittel und/oder Brausehilfsmittel granuliert und dann mit einem wasserlöslichen aber als Wasserdampfsperre wirkenden Filmüberzug ummantelt wird.

10. Verfahren gemäß Anspruch 9, **dadurch gekennzeichnet, daß** mit einer wäßrigen Dispersion von Vitamin E und/oder ungesättigten Fettsäuren granuliert wird.

11. Verfahren gemäß Anspruch 10, **dadurch gekennzeichnet, daß** nach dem Granulieren aber vor der Ummantelung mit dem Filmüberzug abgestäubt wird.

12. Verfahren gemäß Anspruch 11, **dadurch gekennzeichnet, daß** mit feinteiligen Magnesiumlaktat abgestäubt wird.

## Claims

1. A bath salt having a magnesium salt content, **characterized in that** it is provided as a flowable, nonhygroscopic granulate and said granulate consists of cores containing at least 30 % by weight of magnesium chloride crystals but no substantial amounts of calcium salts, wherein the cores of the granulate are sheathed by a water-soluble film coating which, however, acts as a water vapour barrier.

2. The bath salt according to claim 1, **characterized in that** it additionally contains vitamin E and/or unsaturated fatty acids.

3. The bath salt according to claim 1 or 2, **characterized in that** it contains disintegrants and/or effervescent auxiliaries.

4. The bath salt according to claim 2 or 3, **characterized in that** said disintegrant and/or said effervescent auxiliaries are provided in the cores of the granulate and said vitamin E and/or said unsaturated fatty acids are provided as a film layer between the cores of the granulate.

5. The bath salt according to any one of claims 1 to 4, **characterized in that** the magnesium chloride content exceeds 50 % by weight.

6. The bath salt according to any one of claims 1 to 5, **characterized in that** the magnesium chloride content is from 55 to 95 % by weight.

7. The bath salt according to any one of claims 2 to 6, **characterized in that** the vitamin E and/or unsaturated fatty acid content is from 0.5 to 10 % by weight.

8. The bath salt according to any one of claims 3 to 7, **characterized in that** the disintegrant and/or effervescent auxiliary content is from 2 to 10 % by weight.

9. A process for manufacturing the bath salt according to claim 1, **characterized in that** crystalline magnesium chloride is granulated optionally in admixture with a disintegrant and/or effervescent auxiliary and then sheathed by a water-soluble film coating which, however, acts as a water-vapour barrier.

10. The process according to claim 9, **characterized in that** an aqueous dispersion of vitamin E and/or unsaturated fatty acids is used in the granulation.

11. The process according to claim 10, **characterized in that** a dusting is performed subsequent to the granulation but prior to the sheathing by said film coating.

12. The process according to claim 11, **characterized in that** finely divided magnesium lactate is used for dusting.

## Revendications

1. Sel de bain contenant des sels de magnésium, **caractérisé en ce qu'**il se présente sous la forme d'un granulat apte à écouler librement, non hygroscopique, et **en ce que** ce granulat est constitué de noyaux contenant au moins 30 % en poids de cristaux de chlorure de magnésium, sans quantité importante de sels de calcium, et dans lequel les grains du granulat sont enrobés d'une couche superficielle qui est soluble dans l'eau, mais qui agit comme une barrière vis à vis de la vapeur d'eau.

2. Sel de bain selon la revendication 1, **caractérisé en ce qu'**il contient en plus de la vitamine E et/ou des acides gras insaturés.

3. Sel de bain selon la revendication 1 ou 2, **caractérisé en ce qu'**il contient un agent de mouillage et/ou des agents effervescents.

4. Sel de bain selon la revendication 2 ou 3, **caractérisé en ce que** l'agent de mouillage et/ou les agents effervescents sont présents dans les noyaux des grains du granulat et la vitamine E et/ou les acides gras insaturés forment une couche superficielle entre les noyaux du granulat.

5. Sel de bain selon une des revendications 1 à 4, **caractérisé en ce que** la teneur en chlorure de magnésium est supérieure à 50 % en poids.

6. Sel de bain selon une des revendications 1 à 5, **caractérisé en ce que** la teneur en chlorure de magnésium est de 55 à 95 % en poids.

7. Sel de bain selon une des revendications 2 à 6, **caractérisé en ce que** la teneur en vitamine E et/ou en acides gras insaturés est de 0,5 à 10 % en poids.

8. Sel de bain selon une des revendications 3 à 7, **caractérisé en ce que** la teneur en agent de mouillage et/ou en agent effervescent est de 2 à 10 % en poids.

9. Procédé de préparation d'un sel de bain selon la revendication 1, **caractérisé en ce qu'**on fabrique des granulés avec du chlorure de magnésium cristallin éventuellement en mélange avec un agent de mouillage et/ou un agent effervescent et qu'ensuite on l'enrobe d'une couche superficielle soluble dans l'eau, mais agissant comme une barrière vis à vis de la vapeur d'eau.

10. Procédé selon la revendication 9, **caractérisé en ce que** la granulation s'effectue avec une dispersion aqueuse de vitamine E et/ou d'acides gras insaturés.

11. Procédé selon la revendication 10, **caractérisé en ce qu'**après la granulation, mais avant l'enrobage par la couche superficielle, on effectue un saupoudrage.

12. Procédé selon la revendication 11, **caractérisé en ce qu'**on effectue un saupoudrage avec du lactate de magnésium finement dispersé.
